# EUROPEAN PATENT SPECIFICATION

(11) **EP 4 440 524 B1**
(45) Date of publication and mention of the grant of the patent: **12.08.2026**
(21) Application number: 22830193.3
(22) Date of filing: 02.12.2022
(51) Int. Cl.: A61F 13/15, A61F 13/532, A61F 13/537, D04H 1/435, D04H 1/559, D04H 1/70

(54) **ENVIRONMENTALLY-FRIENDLY ABSORBENT ARTICLE AND PROCESS OF MAKING**
UMWELTFREUNDLICHER ABSORBIERENDER ARTIKEL UND VERFAHREN ZU SEINER HERSTELLUNG
ARTICLE ABSORBANT SANS DANGER POUR L'ENVIRONNEMENT ET SON PROCÉDÉ DE FABRICATION

(30) Priority: 03.12.2021 EP 21212364
(43) Date of publication of application: 09.10.2024
(73) Proprietor: Ontex BV, 9255 Buggenhout (BE); Ontex Group NV, 9320 Erembodegem (BE)
(72) Inventor: COPPEJANS, Toon, 9270 Laarne (BE); IDELSON, Alissa, 53359 Rheinbach (DE); WEBER, Ainas, 53474 Bad Neuenahr-Ahrweiler (DE); COBBAERT, Dries, 1770 Liedekerke (BE)
(74) Representative: Macchetta, Andrea
(86) International application number: PCT/EP2022/084307
(87) International publication number: WO 2023/099778

(56) References cited:
- EP-A1- 3 466 387
- WO-A1-2016/108039
- GB-A- 2 263 914
- JP-A- 2002 159 533
- US-A1- 2016 175 169
- US-A1- 2021 290 455

## Description

### TECHNICAL FIELD

The disclosure relates to absorbent articles such as disposable absorbent articles, preferably selected from the group consisting of diapers (whether for baby or adults), pants (whether for baby or adults), briefs, and combinations thereof and the methods of making thereof.

### BACKGROUND

Disposable absorbent articles, such as diapers, are designed to contain bodily wastes and prevent soiling of the wearer's clothing and/or other items (e.g., a bed, a chair, a blanket, etc.). The absorbing and anti-leakage properties of such articles is generally important. Such articles are also designed to be cost-effective, and therefore manufacturers generally make the articles applicable for use by individuals with a wide range of body types. Accordingly, new and improved disposable absorbent articles that both conform to a wide range of body types and fit snuggly to the user to contain wastes and limit leakage whilst being even more economical and environmentally-friendly are of continued interest.

One way that manufacturers have attempted to address the continued interest is by reducing the amount of absorbent material by the introduction of channels that aid and promote liquid distribution along and across the absorbent core. Examples include those as described in EP 3 342 386 B1, EP 3 542 766 B1, EP3 562 454 B1, EP3 692 963 B1, and EP3 562 455 B1.

In other examples cores that eliminate the fluff content have been attempted that immobilise superabsorbent polymer particles on nonwoven substrates such as described in EP 3 085 346 B1, and EP 3 348 246 A1.

In other more recent examples, cores that comprise a lofty layer onto which superabsorbent polymer particles are deposited and immobilised by adhesive are contemplated such as described in EP 3 881 814 A1.

US20160175169 A1 relates to an absorbent core for use in an absorbent article, the absorbent core extending in a transversal direction and a longitudinal direction, the absorbent core comprising: a fluid-permeable top layer, a bottom layer, and a central layer sandwiched between the top layer and the bottom layer the central layer is a high loft fibrous nonwoven layer, in particular having a density of less than 0.200 g/cc, measured at a pressure of 4.14 kPa. Superabsorbent polymer particles are blended with the fibers of the central layer, except for one, two or more longitudinally-extending channels substantially free of superabsorbent polymer particles. Document WO2016/108039A1 discloses further relevant prior art related to the present disclosure.

Thus, although there have already been significant advancements in technology in this field, there still remains a need to further improve core constructs and articles that may enable the reduction of adhesive used and overall improve thus its cost as well as environmental impact whilst still retaining the overall performance (especially in terms of absorption, rewet and leakage prevention) normally associated with todays' premium absorbent articles.

### SUMMARY

In a first aspect, the invention relates to an absorbent core for use in an absorbent articles, as defined in Claim 1.

In another aspect, the disclosure relates to an absorbent article comprising a liquid permeable topsheet, a liquid impermeable backsheet and an absorbent core sandwiched therebetween, wherein the absorbent core is as described hereinabove.

In another aspect, the disclosure relates to a method for the manufacture of an absorbent core comprising the steps of: (i) providing a first, preferably substantially continuous, nonwoven web having a first void volume; (ii) selectively applying heat to said first nonwoven web such that a deposition area of said first nonwoven web increases its void volume to a second void volume that is greater than said first void volume; (iii) depositing absorbent material comprising, preferably consisting of, superabsorbent particles onto the deposition area of said first nonwoven web; (iv) passively or actively cooling the deposition area of said first nonwoven web so that the void volume reduces to substantially the first void volume; (v) optionally combining the first nonwoven web with second and third nonwoven webs such that said first nonwoven web is interposed between said second and third nonwoven webs preferably by joining said webs by mechanical bonding selected from hot pressing and/or ultrasonic bonding; wherein the first nonwoven web consists of a high loft fibrous nonwoven layer that is free of cellulose fibers; said high loft fibrous nonwoven layer comprising, preferably consisting of, a carded air-through bonded nonwoven or drylaid thermobonded nonwoven.

### BRIEF DESCRIPTION OF THE FIGURES

**Fig. 1** Illustrates a planar view according to an embodiment of absorbent cores herein having layers partially removed.
**Fig. 2** Illustrates a planar view according to an embodiment of absorbent cores herein having layers partially removed.
**Fig. 3** Illustrates a cross-section view taken about axis T-T of Fig.1.
**Fig. 4** Illustrates a planar view according to an embodiment of absorbent articles herein having layers partially removed.
**Fig. 5** Illustrates an exemplary process for the production of high-AUL Bio-SAP.
**Fig. 6** Is a schematic diagram illustrating a method of making absorbent cores according to an embodiment herein.
**Fig. 7** Illustrates a top plan view of a first nonwoven web according to an embodiment herein.
**Fig. 8** Illustrates a cross-section similar to Fig.3 according to an embodiment of absorbent cores herein.
**Fig. 9** Illustrates a schematic representation of rugosities according to an embodiment herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Adhesive" as used herein refers broadly to a material, which may or may not be flowable in solution or when heated, that is used as primary function to bond materials or layers together. This may include for example hotmelt adhesives, UV-activatable adhesives, pressure-sensitive adhesives, solvent-based adhesives, and the like.

"Hotmelt adhesive" as used herein refers to a specific class of adhesives and is a form of thermoplastic adhesive.

The term "mechanical bonding" as used herein generally means the joining of one or more layers and/or components preferably by means selected from the group consisting of ultrasonic bonding, thermal bonding, pressure bonding, and combinations thereof.

The terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding (or air-through bonding), wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

The term "high loft" refers to low density bulky fabrics, as compared to flat, paper-like fabrics. High loft webs are characterized by a relatively high porosity. This means that there is a relatively high amount of void space between the fibers in which superabsorbent polymer particles can be distributed and/or impregnated. The high loft layer (without the superabsorbent particles) of the invention may have a density at a pressure of 4.14kPa (0.6 psi) below 0.20 g/cm3, in particular ranging from 0.01 g/cm3 to 0.20 g/cm3, or from 0.05 g/cm3 to 0.15 g/cm3, or from 0.0.10 g/cm3 to 0.14 g/cm3. The high loft layer (without the superabsorbent particles) of the invention may have a density at a pressure of 2.07 kPa (0.3 psi) below 0.20 g/cm3, in particular ranging from 0.05 g/cm3 to 0.15 g/cm3, or from 0.08 g/cm3 to 0.13 g/cm3. The high loft layer (without the superabsorbent particles) of the invention may have a density at a pressure of 0.83 kPa (0.12 psi) below 0.15 g/cm3, in particular ranging from 0.01 g/cm3 to 0.15 g/cm3, or from 0.05 g/cm3 to 0.12 g/cm3 or from 0.08 g/cm3 to 0.10 g/cm3. The density can be calculated by dividing the basis weight of the high loft layer by its thickness measured at the respective pressure as indicated (a method according to ASTM D1777 WSP 130.1(09) may be used for such measurements, and the measurements are preferably carried out on the raw material prior to superabsorbent particle impregnation i.e. the pre-processed raw material and/or the raw material as delivered by a supplier).

"Curing" as used herein typically refers to a process by which resins, binders and/or plastics (and/or UV-activatable and/or heat-activatable adhesives as described herein) are set into or onto fabrics (preferably nonwoven layer or substrate), usually by heating and/or light (and/or UV), to cause them to stay in place and/or provide fixation and/or adhesion between materials and/or layers; the setting may occur by removing solvent or by cross-linking so as to make them insoluble.

The term "absorbent article" refers to a device that absorbs and contains liquid, and more specifically, refers to a device that is placed against or in proximity to the body of the wearer to absorb and contain the various wastes/exudates discharged from the body.

The term "attached" or "adhered" "or joining" refers to elements being connected or united by fastening, adhering, bonding, or by any other method suitable for connecting the elements together and to their constituent materials. Many suitable methods for attaching elements together are well-known, including adhesive bonding, pressure bonding, thermal bonding, ultrasonic bonding, mechanical fastening, etc. Such attachment methods may be used to attach elements together over a particular area either continuously or intermittently.

The term "disposable" refers to absorbent articles that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

As used herein, the terms "substantially free of adhesive" or "substantially free of hotmelt adhesive" means less than 15 gsm, preferably less than 10 gsm, more preferably less than 5 gsm, even more preferably less than 2.5 gsm, even more preferably less than 1.5 gsm, even more preferably less than 1 gsm.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

The term "pant" or "pants" refers to an absorbent article generally worn by infants and incontinent persons (whether infants or adults) about the lower torso and that can be applied or removed from the wearer without unfastening. A pant typically comprises a front and back elastic belt portions (or elastic panels) and a crotch portion connecting said belt portions, the belts are typically joined together at lateral seams so as to provide a waste opening circumscribed by the belts and two leg openings circumscribed by the belts and/or crotch portion. The pant may be placed in position on the wearer by inserting the wearer's legs into the leg openings and sliding the pant into position about the wearer's lower torso. While the term "pant" is used herein, pants are also commonly referred to as "closed diapers", "prefastened diapers", "pull-on diapers", "training pants" and "diaper-pants".

"Passively cooling" as used herein means the cooling of a material and/or substrate in ambient conditions typically meaning that there is no driven cooling source and the cooling is achieved by the temperature and relative humidity in the ambient air surrounding the material and/or substrate being processed. Passive cooling is typically carried out at a temperature of about 25°C to 30°C and generally a relative humidity of generally about 50%.

"Actively cooling" as used herein means the cooling of a material and/or substrate by a driven cooling source. The driven cooling source may be a cooling fan or refrigerant chamber. Active cooling may also or alternatively be achieved by other means, such as by using a pressure chamber whereby the pressure in the chamber is decreased compared to the ambient pressure outside of the chamber so that the temperature within the chamber is reduced. The temperature in the active cooling step is typically from about 10°C to about 25°C. The active cooling may further comprise a dehumidifier such that the relative humidity in the active cooling step is from about 5% to about 40%, preferably from 10% to 30%.

The terms "releasably attached," "releasably engaged," and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.

Embodiments of the articles and processes according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT CORE

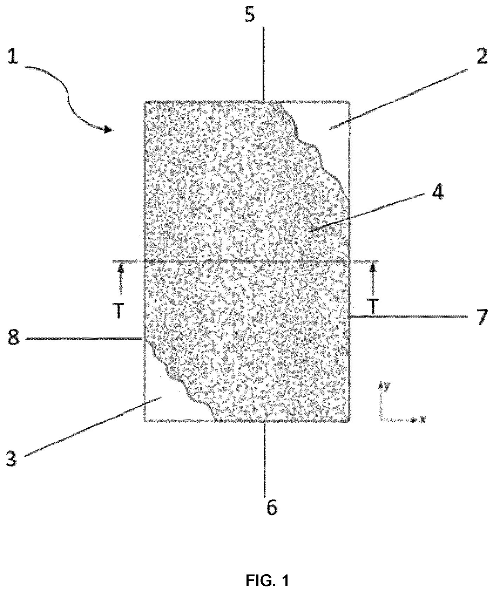

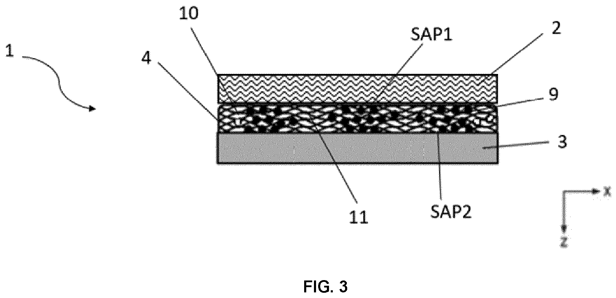

Referring to exemplary Figs. 1-3, absorbent cores (1) herein for use in an absorbent articles (12), typically extend in a transversal direction (x) and a longitudinal direction (y), and may comprise: a fluid-permeable top layer (2), being generally body-facing; a bottom layer (3), being generally garment-facing compared to the top layer; one or more central layers (4) sandwiched between the top layer (2) and the bottom layer (3), each central layer having a front edge (5), a back edge (6) and two longitudinally extending side edges (7, 8), wherein the central layer (4) is a high loft fibrous nonwoven layer which is free of cellulose fibers; wherein the high loft fibrous nonwoven layer comprises, preferably consisting of, a carded air-through bonded nonwoven or drylaid thermobonded nonwoven; and wherein the central layer (4) is impregnated with superabsorbent particles (9) such that said particles are comprised through a thickness (z) of said central layer, said thickness extending perpendicular to both the transversal direction (x) and a longitudinal direction (y), and wherein the high loft fibrous nonwoven layer is substantially free of hotmelt adhesive, and/or substantially free of adhesive, for immobilizing said superabsorbent particles (9).

In a preferred embodiment, the superabsorbent polymers herein are in the form of elongate fibers. Without wishing to be bound by theory, advantageously this allows for improved absorption per unit weight by leveraging an increase in surface area for fluid acquisition.

In an embodiment, the cores herein comprise stiffening fibers, resilient fibers, and/or absorbent fibers. Preferably at least one of top layer (2), bottom layer (3), and central layer(s) comprise stiffenining fibers (more preferably at least the central layer(s) comprise stiffening fibers). It is worth noting that due to fiber integration, the layers do not require adhesives or latex binders for stability. Additionally, the carded nonwoven of the central layer(s) of the present disclosure can be manufactured from an assortment of suitable fiber types that produce the desired performance characteristics. For example, the central layer(s) may comprise a combination of stiffening fibers, absorbent fibers and resilient fibers.

As will be discussed in more detail hereafter, the types of fibers in the layer(s) of the present disclosure are described in terms of their functionality within the core. For example, absorbent fibers are utilized to absorb liquid insults. Stiffening fibers are utilized to bond together via heat treatment thereby providing stiffness and resiliency to the fluid management layer. Resilient fibers are utilized to provide recovery from compressive forces which act against the fluid management layer.

In order to enhance the stabilizing effect of the integration, crimped, carded fibers may be utilized. One or more of the absorbent fibers, stiffening fibers, and resilient fibers may be crimped prior to integration. For example, where synthetic fibers are utilized, these fibers may be mechanically crimped via intermeshing teeth. As for the absorbent fibers, these fibers may be mechanically crimped and/or may have a chemically induced crimp due to the variable skin thickness formed during creation of the absorbent fibers.

The amount of absorbent fibers can impact the absorption of liquid insults. However, when absorbent fibers absorb liquid, they tend to lose some of their structural integrity. The loss of structural integrity can reduce the resiliency of the absorbent article and lead to increased bunching and increased leakage. Accordingly, while in principle, a large percentage of absorbent fibers is good for draining liquid insults from the wearer-facing surface and/or topsheet rapidly, a large percentage can also lead to other problems with the absorbent article.

Typically the layer(s) herein may comprise from about 10 percent to about 60 percent by weight, from about from about 15 percent to about 50 percent by weight, from about 20 percent to about 40 percent by weight, specifically including any values within these ranges and any ranges created thereby of absorbent fibers.

Stiffening fibers may be utilized to help provide structural integrity to the fluid management layer. The stiffening fibers can help increase structural integrity of the fluid management layer in a machine direction and/or in a cross-machine direction which can facilitate web manipulation during processing of the fluid management layer for incorporation into a disposable absorbent article.

Some suitable linear density values of stiffening fiber are provided. For example, the stiffening fiber linear density may range from about 1.0 dtex to about 6 dtex, from about 1.5 dtex to about 5 dtex, or from about 2.0 dtex to about 4 dtex, specifically reciting all values within these ranges and any ranges created thereby. In another specific example, the dtex of the stiffening fibers is about 2.2 dtex.

Some examples of suitable stiffening fibers include bi-component fibers comprising polyethylene and polyethylene terephthalate components or polyethylene terephthalate and co-polyethylene terephthalate components. The components of the bi-component fiber may be arranged in a core sheath arrangement, a side by side arrangement, an eccentric core sheath arrangement, a trilobal arrangement, or the like. In one specific example, the stiffening fibers may comprise bi-component fibers having polyethylene/polyethylene terephthalate components arranged in a concentric, core-sheath arrangement where the polyethylene is the sheath.

While other materials may be useful, the stiffness of polyethylene terephthalate is useful in creating a resilient structure. In contrast, the polyethylene component of the stiffening fibers can be utilized to bond to one another during heat treatment. This can help provide tensile strength to the web in both the MD and CD. Additionally, the bonding of the polyethylene component to other polyethylene components of stiffening fibers can create fixed points in the nonwoven. These fixed points can reduce the amount of fiber-to-fiber sliding which can increase the resiliency of the material.

One of the benefits of the stiffening fibers is that the integrated nonwoven may be heat treated post fiber entanglement. The heat treatment can provide additional structural integrity to the integrated nonwoven by forming bonds between adjacent stiffening fibers. So, where there is a higher percentage of stiffening fibers, more connection points may be created.

Preferably, the central layer (4) comprises a void volume that is greater than the void volume of the fluid-permeable top layer, preferably wherein the top layer has an average pore size and/or void volume that are smaller than the average diameter of the superabsorbent particles. The bottom layer may also have an average pore size (herein meaning typically the average diameter thereof) and/or void volume that are smaller than the average diameter and/or volume of the superabsorbent particles. Advantageously this allows to retain the superabsorbent particles such that they are restrained from migrating towards the upper surface in contact with a subject's skin (or towards the bottom surface in contact with the impermeable backsheet to thus limit the risk of puncturing thereof and leakage), moreover may be beneficial in further limiting rewet.

In an embodiment, the superabsorbent particles (9) are mechanically restrained and/or immobilized by a fiber network (10) of the high loft fibrous nonwoven layer. This allows for reducing the amount of adhesive for immobilisation of the superabsorbent particles.

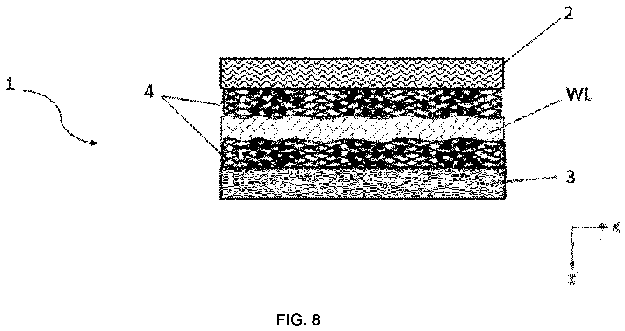

Referring to Fig.8, in an embodiment, the absorbent cores (1) herein comprise more than one central layer (4) wherein at least a first central layer is stacked over and/or above at least a second central layer. Preferably each of the central layers are impregnated with superabsorbent particles, generally as described herein, and wherein a further wicking layer (WL) is interposed between the first central layer and the second central layer preferably such that liquid that is not absorbed by the first central layer comes into fluid communication with said wicking layer that acts as an intra-core distribution layer so as to generally spread and conduct liquid to the second central layer. In this embodiment, it is preferred that the first central layer comprises superabsorbent particles that consist essentially of SAP1 (as described herein below) and the second central layer comprises superabsorbent particles that consist essentially of SAP2 (as described herein below), and wherein the first central layer is positioned above the second central layer such that it is closer to the top layer than said second central layer. The wicking layer herein may be a nonwoven selected from the group of drylaid, airlaid, wetlaid, spunlaid, meltblown and combinations thereof, preferably a wetlaid or airlaid nonwoven. In an embodiment, the wicking layer is a nonwoven comprising thermal bonding selected from calendaring; or mechanical bonding selected from needle-punching or hydro-entanglement, preferably hydro-entanglement. In a preferred embodiment the wicking layer is a spunlace nonwoven. In a highly preferred embodiment the wicking layer is free of synthetic fibers and typically comprises cellulose or cross-linked cellulose fibers that may be part of the nonwoven structure and/or comprised as staple fibers. In an embodiment, the wicking layer comprises hemp fibers. Advantageously such wicking layers not only aid fluid distribution but further provide a further barrier to superabsorbent particle migration between central layers. Moreover, since the wicking layer is positioned between central layers (away from a subject's/wearer's skin), nonwovens that typically have been less desirable due to high rewet (albeit having good fluid transport abilities) now become desirable in applications herein, as well as allowing for more sustainable executions free of synthetic fibers.

Wicking layers herein may have a basis weight of from 10 gsm to 50 gsm, preferably from 15 gsm to 45 gsm, even more preferably from 18 gsm to 40 gsm, even more preferably from 19 gsm to 35 gsm.

In a preferred embodiment, the wicking layer comprises a plurality of rugosities (typically in the form of a plurality of macro-peaks-and-valleys). The rugosities are typically formed at least on the outermost top and/or bottom faces of the wicking layer and may be formed by embossing of the wicking layer with one or more patterns (e.g. a wave-like or ridge-like pattern) and/or by creating densified regions of fibers and non-densified regions of fibers for example by other mechanical means such as variable speed lamination with pull and/or push along the machine direction and/or cross machine direction. The densified regions in a substrate may have a thickness that is less than about $\frac{1}{2}$, preferably less than about $\frac{1}{3}$, of the thickness of the non-densified areas of the substrate, for example. Each rugosity may be described to have a crest (C) at its highest point and a trough (T) at its lowest point and may comprise a rugosity length, a rugosity amplitude and a rugosity frequency. The rugosity length is generally defined by the linear distance in the machine direction (MD) between two adjacent crests; the amplitude (A) is generally defined by the linear distance in the z direction between an adjacent crest (C) and a trough (T); and the frequency (F) is generally defined by the rugosity length per a unit length, resulting as the number of rugosities per cm. Measurements are typically taken by light microscopy with image analysis. A suitable instrument is a HIROX Microscope (Model KH7700) fitted with Adapter OL-35 and lens MXG 10-C or equivalent. An external white light source may be used. Images are acquired and analyzed with HIROX software (Version 2.10C) or equivalent 3D image analysis software. The sample to be tested is preferably preconditioned at 23° C.±2 C.° and 50%±2% relative humidity for 2 hours prior to testing.

When the wicking layer is a nonwoven comprising hydroentangled fibers, it is preferable that the rugosities comprise densified regions of fibers and non-densified regions of fibers that may be formed by use of a patterned perforated plate onto which the fibers are deposited prior to the water jets being applied in the hydroentanglement bonding process step. For example the patterned perforated plate may comprise a plurality of openings that are aligned to form alternating first and second zones of openings wherein the first zones comprise openings that are closer together and/or have a smaller diameter than the second zones. The first and second zones may be arranged in a repeated alternating pattern forming substantially continuous substantially straight or sinusoidal shapes (i.e. the openings collectively having similar characteristic e.g. closer together or smaller size, will together be arranged in a substantially continuous substantially straight or sinusoidal lines/arrays). In such manner the resulting nonwoven may comprise a plurality of alternating wave-like pattern of rugosities that may advantageously be used as wicking layers herein.

In an embodiment, the wicking layer (WL) comprise a nonwoven selected from the group consisting of a carded air-through bonded nonwoven or drylaid thermobonded nonwoven preferably that has not undergone a void volume increase and is generally substantially free of superabsorbent particles impregnated therein or therethrough. In this embodiment **it is** preferable that the basis weight of the wicking layer (WL) be from $\frac{1}{5}$ to $\frac{1}{2}$, preferably from $\frac{1}{4}$ to $\frac{1}{3}$, of the basis weight of the central layer (4) (the latter measured in absence of superabsorbent particles impregnated therein/therethrough).

The rugosity amplitude (A) may be from 0.5mm to 5.0mm preferably from 0.8mm to 3.5mm; and/or the rugosity frequency (F) may be from 0.5 to 10 rugosities per cm, preferably from 1 to 5 rugosities per cm.

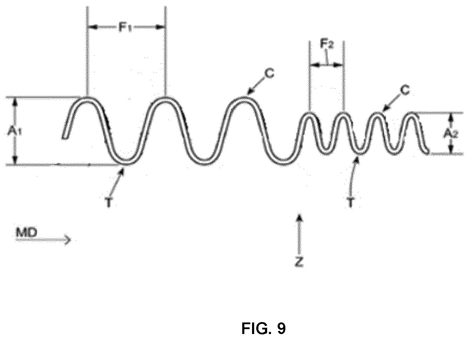

In an embodiment, referring to Fig.9, the wicking layer may have a first plurality of rugosities, having a first amplitude (A₁) and a first frequency (F₁), and a second plurality of rugosities, having a second amplitude (A₂) and a second frequency (F₂); typically wherein the first amplitude (A₁) is greater than the second amplitude (A₂) and/or wherein the first frequency (F₁) is less than the second frequency (F₂). Preferably, the first plurality of rugosities of the wicking layer are positioned to substantially correspond to the deposition area (A_{D}) of the central layer(s) and the second plurality of rugosities are generally positioned outboard thereof (when viewed in a planar direction as described herein). These embodiments advantageously allow the formation of intra-core macro-channels that further aid guiding of the liquid over a larger surface area (when viewed in a planar direction as described herein) between central layers for optimal fast and effective liquid absorption.

Preferably, the high loft fibrous nonwoven layer has been subjected to a void volume decrease from a second void volume, generally corresponding with a superabsorbent particles deposition thereon, to a third void volume, generally corresponding to a state in which the superabsorbent particles have been deposited and impregnated thereon and/or therein, and wherein the third void volume is less, preferably at least 1.2 times less, even more preferably at least 1.5 times less, even more preferably at least 2 times less, than the second void volume. Such improving the mechanical restraining effects on impregnated superabsorbent particles within the fiber network of the high loft fibrous nonwoven layer whilst minimising adhesive usage.

In an embodiment, the superabsorbent particles (9) are impregnated in a pattern comprising one or more areas substantially free of said superabsorbent particles arranged such to form one, two, or more longitudinally extending continuous or discontinuous channels (11) substantially free of superabsorbent particles (9) wherein said channels extend substantially parallel to the longitudinal direction (y). Preferably, the channels (11) are flanked and/or enclosed by superabsorbent particles (9) when viewed in a planar direction wherein the respective plane is formed by the transversal direction (x) and a longitudinal direction (y). The channels are preferably positioned inboard of a perimeter of the core. As further described hereinbelow this permits to form not only fast fluid distribution zones within the core but further creates areas of attachment that may be used for mechanical bonding of the top/bottom layers to the central layer to limit the amount of adhesive used whilst reducing core stiffness and damage to the superabsorbent particles and/or top/bottom layers themselves. Advantageously, this allows for the formation of folding lines to help cup shape formation as well as liquid flow distribution.

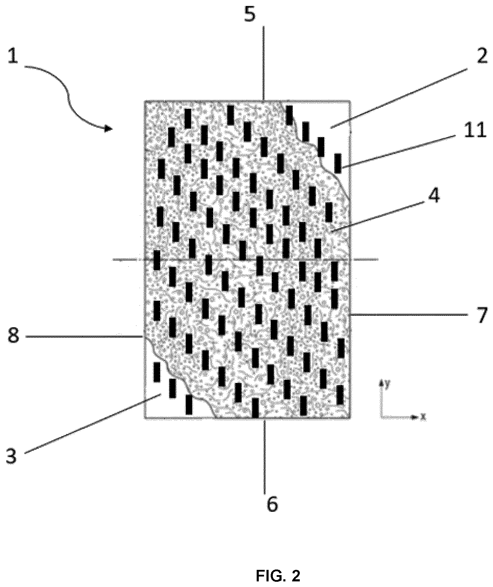

Preferably, as exemplified in Fig. 2, the channels (11) are discontinuous and wherein the sum of the areas (seen from a planar view) total from 10% to 45%, preferably from 15% to 30%, even more preferably from 18% to 25%, of the total (preferably planar) surface area of the absorbent core (1). Typically, the channels (11) are comprised in a plurality of preferably more than 4 (typically from 5 to 50, 10 to 40, and/or 15 to 30) channels and wherein the aspect ratio of said channels (longest dimension divided by smallest dimension) is greater than 1, preferably from 1.2 to 15, more preferably from 1.3 to 10, even more preferably from 1.4 to 8, even more preferably from 1.5 to 5, even more preferably from 1.6 to 3. Typically wherein the longest dimension of the channels is substantially parallel to the longitudinal axis (y). Advantageously this not only allows for the formation of a multitude fast liquid distribution structures between regions with absorbent material (i.e. absence of absorbent material therealong that may otherwise slow down liquid flow by its absorption/expansion effects) but further allows for the formation of joining areas wherein the layers, or nonwoven webs, herein may be bonded together therein by mechanical bonding whilst limiting risks of damaging the SAPs as well as limiting risk of puncturing the layers which would otherwise then allow the SAP to leak through and cause potential piercing on a subject's skin. Preferably the channels are thus arranged in the form of islands within a sea of absorbent material (or superabsorbent particles) typically when viewed in a planar direction as described herein and represented in the figures.

Alternatively, the superabsorbent particles (9) are impregnated in a pattern free of channels (11) substantially free of superabsorbent particles (9). In this embodiment it is preferred to apply a UV-curable adhesive (preferably in powder(or granular)-form or liquid, though if liquid thixotropic liquids may be preferred), preferably at the deposition step or just after as described herein below in the process section. Advantageously this allows for an alternative to hotmelt adhesive and permits to limit or even avoid the overloading the substrate with adhesive (by traditional coating techniques e.g. spraying or slot-coating) on the surface of the central layer (typically at positions closer to the top layer and/or bottom layer) that may impact not only sustainability of the product but also liquid absorption performance due to their typical hydrophobic nature forming a film/barrier or obstacle to liquid vs the discrete network-like structure generally formed by such UV-curable adhesive. When the UV-curable adhesive is a liquid it preferably has a viscosity of greater than 5000 cPS (at 25°C) and preferably a thixotropic index of more than 2 as measured according to ISO/WD 3219-1. UV-curable adhesives for use herein may be selected from modified acrylates and some examples of commercially available adhesives include EA6062, EA6114, U3334, U3349TX manufactured and sold by H.B. Fuller Company, 9001 W. Fey Drive, Frankfort, IL 60423.

In a preferred embodiment the high loft fibrous nonwoven layer is free of hotmelt adhesive and preferably free of channels being substantially free of absorbent material.

In an embodiment, the fibers (10) of the high loft fibrous nonwoven layer comprise or consist of a material selected from the group consisting of: polylactic acid or derivatives thereof, polylactic-co-glycolic acid or derivatives thereof, polyhydroxyalkanoates or derivatives thereof, bio-polyethylene, bio-polypropylene, and mixtures thereof, preferably polylactic acid or derivatives thereof. Advantageously, this allows to not only reduce the carbon footprint thereby making the entire absorbent article more environmentally friendly, but further may facilitate the recycling or composting of the used absorbent article. As discussed in more detail below these materials are typically associated with poor softness and its wide use has been somewhat limited up until now due to such performance drawbacks which the inventors have mitigated in central layers and processes herein, especially by void volume increase/decrease as described in more detail herein.

In an embodiment, the central layer (4) is different from the top and bottom layers (2, 3), preferably wherein the top and bottom layers (2, 3) comprise, preferably consist of, a multilayer nonwoven comprising spunbond and/or meltblown fibers. Preferably, wherein the multilayer nonwoven is selected from the group consisting of: SS, SSS, SM, SMS, and SMMS. Advantageously structures with greater mechanical integrity are formed and further may have a lower porosity and/or pore size to mitigate particle migration.

The absorbent cores herein may comprise at least 60%wt of superabsorbent particles, in particular at least 70%wt, preferably at least 80%wt, preferably at least 90%wt, by total weight of the core.

The superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1; SAPs comprising a 100% acrylic acid co-acrylamide with little to no cross link shell, and the like all generally having AUL of less than 20 g/g according to the test method herein.

Preferably, the first superabsorbent particles (SAP1) are free of Low-AUL Bio-SAP. Advantageously this limits rewet drawbacks as described herein.

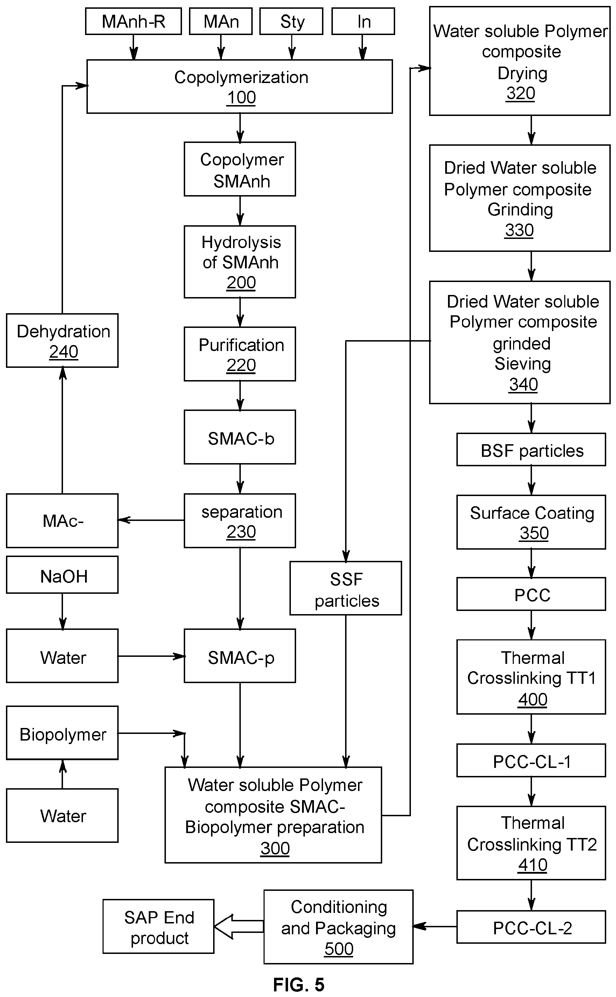

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 5. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb^{®} B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 5, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003- 0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 5. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white color with a value of bulk density of 0.6-0.8 g/cm3. Further , mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis) .

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-I40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi , which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

The first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1/AULSAP2) of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

Suitable central layers for use herein may be for example a triple-layer nonwoven having basis weight of from 55 to 150 g/m² having:
- a first layer corresponding to from 15-25% of the weight (e.g. 37.5 gsm) and consisting in a blend of fibers (as described herein) having a titer between 2 dtex and 28 dtex;
- a second layer corresponding to from 15-25% of the weight (e.g. 37.5 gsm) and consisting in a blend of fibers (as described herein) having a titer between 0 dtex and 7 dtex, and
- the third layer corresponding to from 50-70% of the weight (e.g. 75 gsm) and consisting in a blend of fibers (as described herein) having a titer between 2 dtex and 28 dtex.

Without wishing to be bound by theory, the capability of a material to increase/decrease its void volume is generally dependent on at least one of a chemical composition of the material, a spatial shape (of the fibres and/or its bonding pattern) of the material, a curvature (of the fibres) of the material, type of fibres, composition of fibres. Preferably, the central layer comprises at least two types of fibres being (i) binder fibres and (ii) carrier fibres. The binder fibres may be for example sheath-core bicomponent fibres and the carrier fibres may be for example bio-PE fibres.

In a preferred embodiment, the high loft fibrous nonwoven layer comprises, preferably consists of, a blend of fibers and/or bicomponent fibers (such as binder fibers and carrier fibers as described herein) of a material selected from the group consisting of: polylactic acid (PLA) or derivatives thereof, polylactic-co-glycolic acid (PLGA) or derivatives thereof, polyhydroxyalkanoates (PHA) or derivatives thereof, bio-polyethylene (bio-PE), bio-polypropylene (bio-PP), and mixtures thereof. For example the fibers may be selected from bio-PE/bio-PP, PLA/PP, PLA/bio-PP, PLA/PHA, PLA/PLGA, PHA/PLGA, PLA/bio-PE, PHA/bio-PP, and PLGA/bio-PP, and/or combinations thereof. Particularly PLA and blends thereof is preferred for its sustainable nature, though traditionally has been associated with poor softness particularly exacerbated in highly adhesive loaded structures. Advantageously, the use of PLA and blends within the central layers herein and the reduced usage of adhesive allows for a pliable structure that is sustainable and further does not provide roughness or softness-impairing consequences onto a wearer's skin.

In an embodiment, the central layer(s) comprise a void volumes that is greater than the void volumes of the fluid-permeable top layer, preferably wherein the top layer has an average pore size and/or void volumes that are smaller than the average diameter of the superabsorbent particles. Typically, the central layer(s) are porous layers, wherein porous herein is defined by a void volume ranging from about 300 to about 500 cm3 of void volume/m2, whereas the top layer is comprised of fine hydrophilic fibers, ranging from 0.7 to 30 dtex and preferably from 1.5 to 7 dtex, resulting in small voids and thereby hindering liquid from going back to the surface, and also limiting the possibility for the superabsorbent particles to migrate therethrough.

The void volumes are related to the space between the fibers, which are bound together at multiple points, thereby forming an array presenting cavities, or voids. The void volume in a nonwoven material is a parameter well known to the person skilled in the art, and corresponds to all space available in a material which is not filled by material, like fibers and SAP. Calculation and measurement can be made by PMI porosimetry or air permeability. Preferably, the void volume, measured via air permeability at 100 Pa-20 cm2, corresponds to an air permeability of between 1000 I/m2/s and 12,000 I/m2/s, still preferably between 2000 I/m2/s and 3000 I/m2/s.

The top layer and the bottom layer are typically a nonwoven or a tissue paper, the bottom layer may however be a film and may be liquid impermeable though preferably is a nonwoven. Low basis weight tissue paper for example is readily available and a relatively cheap substrate. The absorbent core may also comprise a wrapping layer (or core wrap) that completely covers and/or envelopes the bottom layer or top layer and may form a C-wrap, sandwich wrap, or G-fold wrap, around the longitudinally extending side edges of the central layer(s) to at least partially cover the top layer or bottom layer respectively and provide better containment of the superabsorbent particles within the absorbent core thus preventing losses of particles especially on the side edges of the core. Alternatively, such a core wrap may also be formed by the top layer or the bottom layer.

The absorbent cores herein may also comprise a multi-layer construction comprising a first central high loft layer and a second (or more) central high loft layer. This construction may provide additional benefits for example in terms of a higher quantity of superabsorbent particles may be distributed within multi-layers as well as allowing the blend of even more superabsorbent polymer grades and/or types between the said layers for optimal liquid absorbency performance through the thickness of the core. These and other optional features of the invention will be described herein.

The thickness, basis weight and density of the central layer are typically substantially homogenous in both transversal direction (x) and longitudinal direction (y). The orientation of fibers in the central layer are typically in-homogenous such as predominant orientation of fibers into one direction x or y such as is the case in carded nonwovens. Furthermore, the fiber orientation in the central layer in thickness direction z may be different versus the predominant orientation in one or both directions x and/or y. The high loft layer may in particular have a thickness of at least 0.30 mm, in particular ranging from 0.35 mm to 2.00 mm, or from 0.50 mm to 1.5 mm, as measured at a pressure of 4.14 kPa (0.6 psi) (a method according to ASTM D1777 NWSP 120.2(09) may be used accordingly for such measurements although a person skilled in the art would understand that other less preferred methods may be used such as caliper measurements via an SDL portable thickness gauge Model no 258b (0.01mm graduation) or equivalent; such method is preferably carried out for the nonwoven in absence of entrained superabsorbent particles e.g. directly from the unused raw material prior to superabsorbent particle entrainment/deposition). The high loft layer may in particular have a thickness ranging from 0.30 mm to 2.50 mm or from 0.5 to 2.0 mm or from 0.7 to 1.3 mm, as measured at a pressure of 0.83 kPa (0.12 psi) (similarly to the above, a method according to ASTM D1777 NWSP 120.2(09) but at the adjusted pressure may be used; such method is preferably carried out for the nonwoven in absence of entrained superabsorbent particles e.g. directly from the unused raw material prior to superabsorbent particle entrainment/deposition). The basis weight of the high loft central layer may for example range from 15 gsm to 500 gsm, preferably from 30 gsm to 200 gsm, for example between 55 gsm and 150 gsm. The values indicated herein for the central layer are considered for the high loft material taken in isolation, that is before the superabsorbent particles have been deposited between the fibers. When the absorbent core comprises two or more high loft central layers, these may be the same or different.

The central layer (or layers) herein may generally serve as substrate for the superabsorbent particles which are at least partially distributed within its pores. The superabsorbent particles may be substantially uniformly blended across the thickness of the high loft layer or preferably may be distributed heterogeneously in the vertical and/or thickness direction. Preferably the void volume increase, generally during the heating step (and typically the deposition step) according to processes described herein, comprises a gradient such that the void volume of the central layer at a position closer to the top layer is greater than the void volume of the central layer at a position closer to the bottom layer, and wherein the superabsorbent particles are heterogeneously distributed in the vertical and/or thickness direction, and preferably wherein the basis weight (in g/m²) of superabsorbent particles is greater at a position closer to the top layer than at a position closer to the bottom layer or vice versa. Advantageously, this permits for reduced gel-blocking effects and modulated liquid absorption along the thickness direction of the core. The superabsorbent particles are typically deposited on one side of the nonwoven and drawn into the high loft nonwoven for example by a combination of gravity, negative pressure on the opposite side of the nonwoven, and optionally an active kinetic energy imparting means as will be described in more detail hereinbelow. In this way, some particles remain close to the surface of the high loft central layer and other, typically smaller, particles may penetrate deeper within the fiber network of the high loft nonwoven. The superabsorbent particles which are not trapped within the pores of the high loft but remain at the surface may be further immobilized by forcing them through the fiber network with the kinetic energy imparting means and/or removed by use of removing means such as air-blowing and/or use of a brush. Adhesive is preferably not applied directly on the central layer. Some small amounts of adhesive may be present when combining the top and bottom layers together with the central layer however, application of adhesive when present on the top and bottom layers is preferably applied substantially outboard of the deposition area and/or in a discontinuous pattern typically so as to limit the amount of said adhesive in the deposition area. Preferably the top and bottom layers are joined to the central layer(s) via mechanical bonding such as ultrasonic bonding or other thermo-sealing means as described in more detail herein.

In a highly preferred embodiment, the central layer comprises a deposition area interposed between two, preferably continuously and, oppositely disposed longitudinally extending lateral areas (typically extending along the machine direction and/or flanking said deposition area along longitudinal edges of a perimeter of said central layer) wherein the sum of areas of said lateral areas is less than the area of said deposition area, typically when viewed in a planar direction as described herein and illustrated in the figures. In this embodiment, the deposition area comprises superabsorbent particles through the thickness of the central layer and the lateral areas are substantially free of superabsorbent particles through the thickness of the central layer. Superabsorbent particles may be comprised on a top surface (generally facing the top layer) of the central layer in said lateral areas but generally substantially not in the thickness direction thereof. Advantageously this permits added flexibility and reduced stiffening effects associated with cores highly loaded with superabsorbent particles and further enables the formation of a leakage barrier as described in more detail in embodiments hereinbelow.

Preferably, the deposition area (A_{D}) comprises at least a first zone and at least a second zone wherein the first zone has a first superabsorbent particle basis weight (in g/m²) and the second zone has a second superabsorbent particle basis weight (in g/m²), and wherein the first superabsorbent particle basis weight is greater than the second superabsorbent particle basis weight. Preferably, the first superabsorbent particle basis weight is greater than 250 g/m², more preferably from 300 g/m² to 500 g/m²; and wherein the second superabsorbent particle basis weight is less than 300 g/m², more preferably from 50 g/m² to 250 g/m². Advantageously this not only may allow for benefits in reducing the monolithic-like stiffening effects but further allows for improved localised absorption.

In the above described embodiment, it is preferable that the first and second zones are concentric such that the first zone is substantially contained and/or surrounded by the second zone or vice versa (generally when viewed in a planar direction as described herein and illustrated in the figures). Preferably the first zone has a perimeter that substantially corresponds (in size e.g. mm) to a perimeter of an acquisition distribution layer as for example described hereinbelow. Alternatively or in addition, the first zone may comprise a surface area (generally when viewed in a planar direction) that substantially coincides (in position e.g. one overlaying or stacking over the other) with a surface area of an acquisition distribution layer as for example described hereinbelow.

### THE ABSORBENT ARTICLE

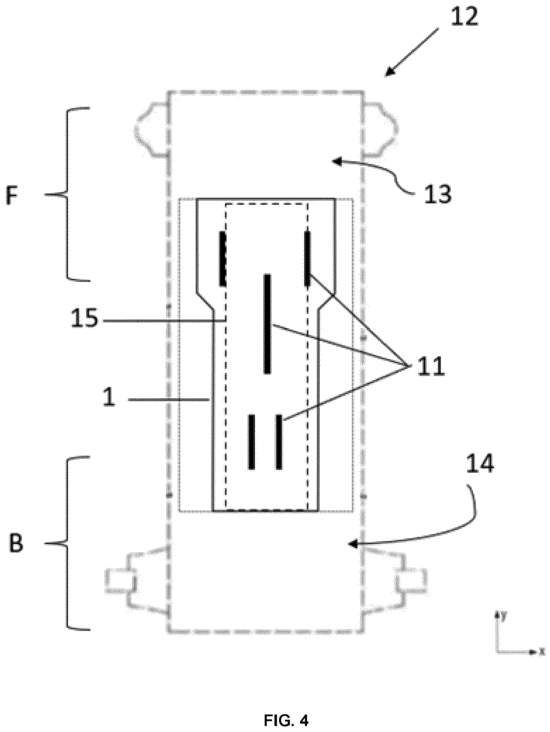

Referring to exemplary Fig. 4, absorbent articles (12) herein generally comprise a liquid permeable topsheet (13), a liquid impermeable backsheet (14) and an absorbent core (1) sandwiched therebetween, wherein the absorbent core (1) is as described hereinabove. Articles herein are preferably selected from the group consisting of disposable diapers and/or pants (including refastenable pants i.e. pants with seams that can be releasably attached). The absorbent article further typically comprising a front portion (F), a back portion (B) and a crotch portion extending therebetween.

Preferably, the absorbent article further comprises an acquisition distribution layer (15) positioned between the topsheet (13) and the absorbent core (1), wherein said acquisition distribution layer (15) is a carded, spunbond and/or spunlaced nonwoven, preferably a carded air-through bonded nonwoven or spunlaced nonwoven; and/or wherein the acquisition distribution layer (15) has a basis weight of from 15 to 55 g/m2 and comprises fibres having an average diameter of from 10 to 35 microns, preferably from 15 to 30 microns, more preferably from 17 to 27 microns.

It is understood that absorbent articles herein may comprise one or more features common is such articles such as one or more of: transversal and/or longitudinal cuffs for leakage prevention; one or more elastic panels and/or belts; one or more waist elastics; one or more male/female closure tapes for fastening the article in an open/closed position; and the like.

### THE PROCESS OF MAKING

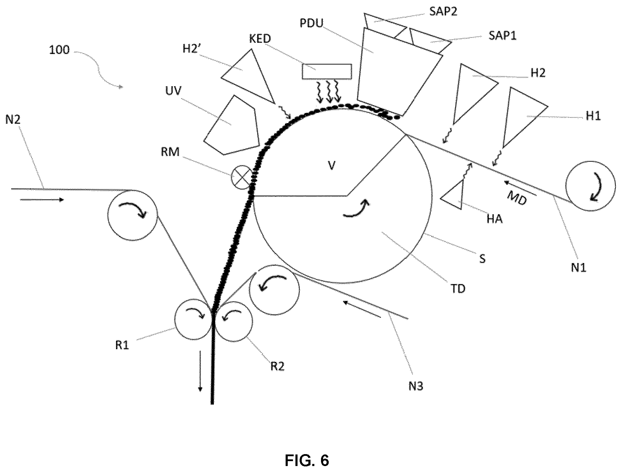

The process for the manufacture of an absorbent cores and/or articles herein, as illustrated in exemplary Fig. 6, typically comprises the steps of: (i) providing a first, preferably substantially continuous, nonwoven web (N1) having a first void volume; (ii) selectively applying heat to said first nonwoven web (N1) such that a deposition area of said first nonwoven web (N1) increases its void volume to a second void volume that is greater than said first void volume; (iii) depositing absorbent material comprising, preferably consisting of, superabsorbent particles onto the deposition area of said first nonwoven web (N1); (iv) passively or actively cooling the deposition area of said first nonwoven web (N1) so that the void volume reduces to substantially the first void volume; (v) optionally combining the first nonwoven web with second and third nonwoven webs (N2, N3) such that said first nonwoven web (N1) is interposed between said second and third nonwoven webs (N2, N3) preferably by joining said webs by mechanical bonding selected from hot pressing and/or ultrasonic bonding; wherein the first nonwoven web (N1) consists of a high loft fibrous nonwoven layer that is free of cellulose fibers; said high loft fibrous nonwoven layer comprising, preferably consisting of, a carded air-through bonded nonwoven or drylaid thermobonded nonwoven. Advantageously such process enables the production of absorbent articles with a reduction in use of adhesive for immobilising the superabsorbent particles.

In a preferred embodiment, the fibers of the high loft fibrous nonwoven layer (as further described herein above) comprises, or consist of, a material selected from the group consisting of: polylactic acid or derivatives thereof, polylactic-co-glycolic acid or derivatives thereof, polyhydroxyalkanoates or derivatives thereof, bio-polyethylene, bio-polypropylene, and mixtures thereof, preferably polylactic acid or derivatives thereof. Advantageously such materials are not only allowing to reduce the content of microplastics and overall improved sustainability impacts, but are further beneficial materials in that they react to heat (i.e. soften) for the desired void volume expansion without necessarily requiring exposure to high temperatures (i.e. also reducing overall production energy usage).

In an embodiment, the first nonwoven web (N1) is different from the second and third nonwoven webs (N2, N3), preferably wherein the second and third nonwoven webs (N2, N3) comprise, preferably consist of, a multilayer nonwoven comprising spunbond and/or meltblown fibers. The multilayer nonwoven may be selected from the group consisting of: spunbond-spunbond (SS), spunbond-spunbond-spunbond (SSS), spunbond-meltblown (SM), spunbond-meltblown-spunbond (SMS), and spunbond-meltblown-meltblown-spunbond (SMMS).

In a preferred embodiment, the superabsorbent particles (as further described herein above) comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Advantageously, SAP1 will tend to stay closer to the top surface of the central layer whilst the SAP2 will tend to impregnate deeper and closer to the bottom surface of the central layer. This further allows for improvements in the balance between absorption speed and reduced rewet whilst mitigating gel-blocking effects that may be particularly undesirable in the absence of cellulose fibers and/or fluff pulp.

Preferably, at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles. More details and advantages are provided in embodiments hereinabove.

The first superabsorbent particles (SAP1) may be comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles. More details and advantages are provided in embodiments hereinabove.

In an embodiment, the AUL ratio (AULSAP1/AULSAP2) of the first superabsorbent particles (SAP1) and second superabsorbent particles (SAP2) is greater than 1.4, preferably greater than 1.5, more preferably from 1.6 to 5, even more preferably from 1.7 to 3. More details and advantages are provided in embodiments hereinabove.

In an embodiment, the second and third nonwoven webs (N2, N3) comprise, or consist of, fibers of a material selected from the group consisting of: polylactic acid or derivatives thereof, polylactic-co-glycolic acid or derivatives thereof, polyhydroxyalkanoates or derivatives thereof, bio-polyethylene, bio-polypropylene, and mixtures thereof, preferably polylactic acid or derivatives thereof. Advantageously the content of non-biodegradable and/or difficult to compost polymers may thus be reduced.

Preferably, the superabsorbent particles are applied in a pattern, such as in the form of at least two spaced apart continuous or discontinuous stripes, so as to form one or more areas that are substantially free of said superabsorbent particles and forming one, two, or more continuous or discontinuous longitudinally extending channels substantially free of superabsorbent particles, preferably wherein said channels extend along an axis that is parallel to a machine direction (MD) and a longest dimension of the first nonwoven web (N1). Preferably (and as discussed in more detail herein above) the channels are discontinuous and comprised in a plurality of preferably more than 4 (typically from 5 to 50, 10 to 40, and/or 15 to 30) channels and wherein the aspect ratio of said channels (longest dimension divided by smallest dimension) is greater than 1, preferably from 1.2 to 15, more preferably from 1.3 to 10, even more preferably from 1.4 to 8, even more preferably from 1.5 to 5, even more preferably from 1.6 to 3. Typically wherein the longest dimension of the channels is substantially parallel to the longitudinal axis (y). Advantageously this not only allows for the formation of fast liquid distribution structures between regions with absorbent material (i.e. absence of absorbent material therealong that may otherwise slow down liquid flow by its absorption/expansion effects) but further allows for the formation of joining areas wherein the layers, or nonwoven webs, herein may be bonded together therein by mechanical bonding whilst limiting risks of damaging the SAPs as well as limiting risk of puncturing the layers which would otherwise then allow the SAP to leak through and cause potential piercing on a subject's skin.

The second and third nonwoven webs (N2, N3), which generally correspond to top and bottom layers (2, 3) described herein, may be joined to the first nonwoven web (N1), which generally corresponds to the central layer(s) (4) described herein, by mechanical bonding selected from hot pressing and/or ultrasonic bonding in the continuous or discontinuous longitudinally extending channels substantially free of superabsorbent particles. This may be done with rotating roller (R1) and opposed counter roller (R2) wherein roller R1 comprises one or more raised elements corresponding to the channel pattern and the opposed counter roller R2 comprises either a smooth outer surface or comprises one or more recesses corresponding to the pattern of raised elements such that they substantially mate with each other when radially aligned. The raised elements may comprise heated elements that come into contact with the nonwoven surface or sonotrodes for contactless ultrasonic welding. Although the use of a roller arrangement has been exemplified, other means may further be contemplated. The top and bottom layers (2, 3), and/or core wrap layers, described herein, are preferably further joined to the first nonwoven web (N1) at the longitudinal side edges (running substantially parallel to the machine direction MD); and preferably further along front and back transverse edges running perpendicular to, and connecting, the longitudinal side edges (to form individual cores in an inline process).

In an alternative, yet preferred, embodiment, the superabsorbent particles are deposited on the first nonwoven web (N1) in a pattern such that substantially no channels free of absorbent material are formed. In this embodiment the superabsorbent particles cover substantially the entirety of at least the deposition area of the first nonwoven web (N1) when seen in a planar view, preferably the weight distribution of the superabsorbent particles is not the same over the deposition area (e.g. more or less particles impregnated in the thickness z direction as described in more detail herein above). In this embodiment, it is preferred to apply an adhesive (typically in powder or liquid form) that is subsequently UV-cured or heat-cured as described in more detail herein. The adhesive may be in powder (or granular) form (at room temperature of about 25°C) and mixed with the superabsorbent particles prior to deposition on the first nonwoven web (N1) so as to generally accurately locate said adhesive where needed for further immobilisation of the superabsorbent polymer particles and joining of the nonwoven webs. Advantageously this allows for significantly reducing the overall amount of adhesive compared to use of hotmelt adhesives by other traditional means such as spraying and/or slot coating so as to attain a central layer or core laminate herein being substantially free of adhesive.

In an embodiment, the deposition step comprises the step of increasing the kinetic energy of the superabsorbent particles by one or more of vibration, electrostatic field, electric field, and/or ultrasonic waves, preferably by a kinetic energy enhancing device (KED), such to enhance absorbent particle penetration through a thickness (z) of the first nonwoven web (N1) that generally extends perpendicular to a width and length of said first nonwoven web (N1) typically wherein the length and width are on a plane perpendicular to the thickness (z) and a particle deposition direction. For example, a Fibroline module with flat electrodes at a speed of from 10 to 30m/min (typically about 20m/min) may be used. Alternatively or in addition, a vacuum (V) may be used to further aid the superabsorbent particles to penetrate and impregnate the first nonwoven web (N1).

Selective heating herein may be applied in various manners by one or more heat application units (H1, H2). For example, the central layer (or first nonwoven web) may be provided on a rotating member or belt and guided through a heating chamber wherein a heating source such as an oven, microwave radiator, infrared lamp, and/or hot air blower(s), produces heat which may be transferred to the central layer (or first nonwoven web). Preferably, a microwave radiator, infrared lamp, and/or hot air blower sized according to the deposition area described herein is used for localized heating of the central layer (or first nonwoven web) in the deposition area.

In an embodiment, especially when the central layer or first nonwoven web (N1) comprises binder fibres and carrier fibres, as the temperature of the central layer or first nonwoven web (N1) is increased the binder fibres typically will begin to soften and some of the binder fibres and/or carrier fibres will position their selves approximately parallel with the softened binder fibres while detaching therefrom. This realigning and/or detaching action will cause the central layer or first nonwoven web (N1) to increase its lofty structure as the void volume thereof increases.

In the exemplified embodiment the apparatus (100) used in methods described herein, typically comprises a first heat application unit (H1) that comprises a first hot air, microwave, and/or infrared device (i.e. heating device), positioned upstream of a particle depositing unit (PDU). Preferably, the upstream first heating device is positioned such that it can apply heat to the central layer or first nonwoven web (N1) before the central layer or first nonwoven web (N1) reaches an outer surface (S) of the transport device (TD) such as a rotating drum former. In this manner, superabsorbent particles can be deposited on the central layer or first nonwoven web (N1) directly after the central layer or first nonwoven web (N1) has been heated by the first heating device. Alternatively, the upstream first heating device is positioned above the outer surface (S) of the transport device (TD) such that it can apply heat to the central layer or first nonwoven web (N1), while the central layer or first nonwoven web (N1) is guided on the outer surface (S) of the transport device (TD), such that superabsorbent particles can be deposited on the central layer or first nonwoven web (N1) substantially concurrently with the central layer or first nonwoven web (N1) being heated by the first heating device. The first heating device is configured to generate and direct heat towards the central layer or first nonwoven web (N1). By directing heat towards the heat sensitive central layer or first nonwoven web (N1), fibres within the central layer or first nonwoven web (N1) will tend to curl under the influence of said heat which causes the central layer or first nonwoven web (N1) to open up. This way, an available volume for accommodating superabsorbent particles which are provided by the particle depositing unit (PDU) within the substrate layer is increased, without damaging the fibers of the central layer or first nonwoven web (N1) and without negatively affecting the wet and/or dry integrity of the absorbent structure. Furthermore, by opening up the fibrous central layer or first nonwoven web (N1) by applying direct or indirect hot air, a deep penetration of superabsorbent particles within the substrate layer may be achieved, and an increased density of superabsorbent particles being distributed within the central layer or first nonwoven web (N1) is made possible, this particularly so when combined with a cooperating vacuum (V) and further kinetic energy enhancing devices (KED) as described herein. The extent to which the available volume within the central layer or first nonwoven web (N1) increases may generally depend on the material of the fibrous central layer or first nonwoven web (N1), the temperature to which the central layer or first nonwoven web (N1) is exposed and the time during which the central layer or first nonwoven web (N1) is exposed to the high temperature.

Typically the drum former as described herein comprises a plurality of pockets about the circumference thereof, wherein each pocket comprises a cavity having the desired shape of each absorbent core that is subsequently laminated and severed such to be contained in each absorbent article made with the process herein. Each pocket typically has a perimeter and/or flange whereby the central layer herein is pocketed sealed and/or enclosed by top/bottom layers and/or a further core wrap as described herein. The flange generally extending along the entire perimeter of the pocket (being both oppositely disposed transverse edges and longitudinal edges connecting said transverse edges). Generally, only the deposition area corresponding to, preferably each, said pocket is loaded with superabsorbent particles, and wherein areas of the deposition area of the first nonwoven web (or central layer) overlapping said flange are free of superabsorbent particles.

**The** temperature that may be used for changing the void volume of central layer(s) or first nonwoven web (N1) as described herein can vary between 45°C and 180°C, but is preferably between 75°C and 125°C.

In an embodiment, the central layer or first nonwoven web (N1) is heated on the top surface thereof and/or both the top and bottom surfaces thereof. When heated on both of the top and bottom surfaces thereof, it is preferable that the heat applied (and/or airflow applied) by an auxiliary heater (HA) is less than that applied by a first heating unit (H1). **The** auxiliary heater (HA) may be positioned substantially parallel or downstream, along a machine direction MD, to the first heating unit (H1). Typically the superabsorbent particle deposition step that generally follows is arranged to deposit superabsorbent particles on the top surface of the central layer or first nonwoven web (N1). Advantageously this allows for an improved void volume expansion rate through the thickness z of the nonwoven and permits to reduce the overall airflow and/or heating temperature needs of each heating unit individually. Moreover a dual-gradient may be formed as fibers are permitted to re-align and loft on multiple axis.

In a preferred embodiment, the first nonwoven web (N1) is heated in at least two sequential stages by at least a first heating unit (H1) and at least a second heating unit (H2) wherein the second heating unit (H2) is positioned downstream of the first heating unit (H1) in the machine direction (MD) and generally upstream the superabsorbent particle deposition unit (PDU). Typically the first temperature applied by the first heating unit (H1) is less than the second temperature applied by the second heating unit (H2), preferably said first temperature being between 30°C and 75°C and the second temperature being between 75°C to 125°C. Advantageously this allows for increased void volume expansion as the fibers are allowed to achieve an increased alignment per unit time without the risk of collapsing by inadvertent heat spots causing excessive melting. Moreover it permits to reduce the overall time that the nonwoven is to be heated hence allowing for higher in-line speeds of more than 4m/s, preferably more than 5m/s, through the heating segments of the apparatus.

In addition or alternatively, the second heating unit or further second heating unit (H2') may be positioned downstream of the superabsorbent particle deposition unit (PDU). In this embodiment, the temperature applied by the further second heating unit (H2') is typically less than the second heating unit (H2) and preferably being between 35°C and 75°C. Preferably, the further second heating unit (H2') is arranged to provide heat to the central layer or first nonwoven web (N1) substantially concurrently to a vacuum (V) that is typically applied by the transport device (TD) providing a pulling force to the particles through the thickness (z) of the central layer or first nonwoven web (N1). Preferably, the further second heating unit (H2') is a hot air blowing device. Advantageously, this arrangement allows to optimize particle penetration by concurrently heating (i.e. substantially preventing void volume contraction), blowing (i.e. pushing) the superabsorbent particles and pulling said particles by said vacuum so as to further aid particle penetration and entrapment.

In embodiments herein where the heating device is a hot air blower, said blower may be typically arranged to provide an air flow rate of from 1m/s to 20m/s, preferably from 2m/s to 15m/s, even more preferably from 3m/s to 10m/s. Although a positive air flow can be beneficial to further aid heat penetration through the central layer or first nonwoven web (N1) thickness (z) too high heat flows may further result in the collapsing of the heated fibers rather than promoting lofty-ing. Moreover, when heat flow is applied downstream the superabsorbent particle deposition step, air flow can be beneficial to provide a pushing force to the particles, yet if too high results in the particles being projected away from the surface of the central layer or first nonwoven web (N1). Preferably air screens are used to maintain the flow substantially laminar.

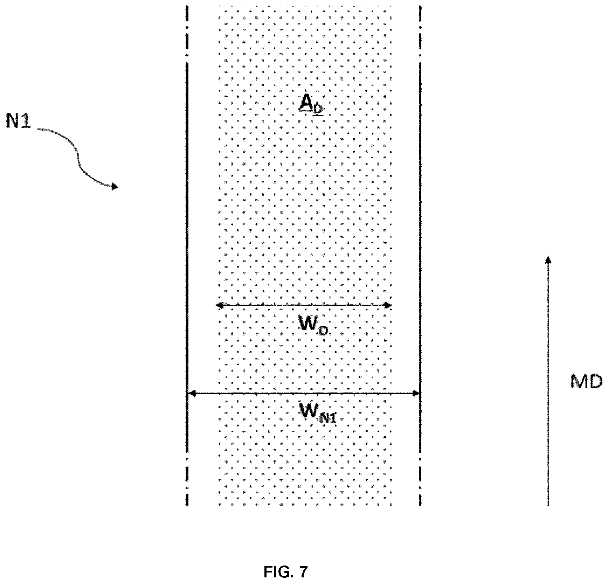

In an embodiment, as exemplified in Fig. 7, the selective heating step is arranged to heat only the deposition area (A_{D}) of said first nonwoven web (N1), and wherein the deposition area (A_{D}) has a width (W_{D}) that is less than the width (W_{N1}) of the first nonwoven web (N1) being substantially perpendicular to the longest length of said web and/or the machine direction (MD), preferably wherein the deposition area has a width that is from 50% to 95% of the width of the web (N1), more preferably from 60% to 90%, even more preferably from 70% to 85%, of the width of the web (N1). Advantageously this allows for the longitudinal edges of the core to substantially not be impregnated (in the z-thickness direction) with superabsorbent particles in the subsequent deposition step allowing on the one hand more flexibility and/or conformity on said edges as well as the possibility to accumulate superabsorbent particles substantially only on the upper surface of said edges so as to form a functional leakage barrier to liquids tending to escape towards the upper edges of the core (in this latter example the superabsorbent particles may further be immobilized by one or more of the techniques described herein).

In an embodiment a UV-activatable adhesive in particulate (or granular) or liquid form (at room temperature of about 25°C) is applied, preferably together with or just after the superabsorbent particles and substantially with the same pattern, to the first nonwoven web followed by curing under UV-light source (UV), preferably wherein said UV-light source (UV) is positioned downstream the kinetic energy enhancing device (KED) and/or particle deposition unit (PDU). Advantageously this allows to avoid the use of hotmelt adhesive and overall energy reduction in-process as well as reduced overall amount as described hereinabove. This for example may be used to further immobilize the superabsorbent particles in the above mentioned embodiment and/or at least partial joining of the second and/or third nonwoven webs (N2, N3) to the first nonwoven web (N1).

In an embodiment a heat-activatable adhesive in particulate (or granular) form (at room temperature of about 25°C) is applied, preferably together with (or just after) the superabsorbent particles and substantially with the same pattern, to the first nonwoven web followed by curing under a heat source, preferably positioned downstream the kinetic energy enhancing device (KED) and/or particle deposition unit (PDU). The heat source may be one of any suitable heat sources described herein. Advantageously this may allow to avoid the use of traditionally higher amounts of adhesive and overall energy reduction in-process as described hereinabove. This for example may be used to further immobilize the superabsorbent particles in the above mentioned embodiment and/or at least partial joining of the second and/or third nonwoven webs (N2, N3) to the first nonwoven web (N1).

In an embodiment a bio-adhesive is applied to one or both the second and third nonwoven webs (N2, N3) in a pattern, preferably comprising continuous or discontinuous stripes, prior to combining with the first nonwoven web (N1) so that at least a portion of said bio-adhesive contacts said first nonwoven web (N1), and wherein the pattern of bio-adhesive substantially corresponds to the pattern of superabsorbent particles so as to further immobilize the superabsorbent particles, preferably wherein the bio-adhesive comprises more than 90%, preferably more than 95%, even more preferably more than 99% bioderived and compostable content. The bio-adhesive may be a polyester based hotmelt adhesive. The bio-adhesive preferably has a softening point according to ASTM D-3461 of from 40°C to 70°C. An exemplary bio-adhesive that may be suitable herein is commercially available Full-Care^{®} Evolution^{™} 5944 manufactured and sold by H.B. Fuller (see hereinabove for further company details).

In an embodiment, the superabsorbent particles which are not trapped within the pores of the high loft but remain at the surface may be removed by use of removing means (RM) such as air-blowing and/or use of a, preferably rotating, brush.

### TEST METHODS

### AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 µm. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as WO. Then 0.900 +/- 0.005 g of water-absorbing polymer or material (particle size distribution 150 - 800 µm or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 µm in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows: $AUL 0.7 psig / g = Wb - Wa / Wa - W 0$

AUL 0.3 psi and 0.5 psi are measured similarly at the appropriate lower pressure.

### Absorbption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

### Equipment and materials:

1. Beaker, 100 ml.
2. Programmable magnetic stir plate, capable of providing 600 revolutions per minute.
3. Magnetic stir bar without rings, 7.9 mm × 32 mm, Teflon covered.
4. Stopwatch.
5. Balance, accurate to ± 0.01 g.
6. Saline solution, 0.9%.
7. Weighing paper.
8. Room with standard condition atmosphere: Temp = 23°C ± 1°C and Relative $Humidity = 50 % \pm 2 % .$

### Test procedure:

1. Measure 50 g ± 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g ± 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented embodiments without reappraisal of the appended claims.

## Claims

1. An absorbent core (1) for use in an absorbent article (12), the absorbent core (1) extending in a transversal direction (x) and a longitudinal direction (y), the absorbent core (1) comprising:
a fluid-permeable top layer (2);
a bottom layer (3);
one or more central layers (4) sandwiched between the top layer (2) and the bottom layer (3), each central layer having a front edge (5), a back edge (6) and two longitudinally extending side edges (7, 8), wherein the central layer (4) is a high loft fibrous nonwoven layer which is free of cellulose fibers;
**characterized in that** said high loft fibrous nonwoven layer consists of, a carded air-through bonded nonwoven or drylaid thermobonded nonwoven; and wherein said central layer (4) is impregnated with superabsorbent particles (9) such that said particles are comprised through a thickness (z) of said central layer, said thickness extending perpendicular to both the transversal direction (x) and a longitudinal direction (y), and **in that** said high loft fibrous nonwoven layer is substantially free of hotmelt adhesive for immobilizing said superabsorbent particles (9) and wherein the superabsorbent particles comprise a blend of superabsorbent particles comprising a first superabsorbent particles and a second superabsorbent particles, wherein the first superabsorbent particles have an AUL that is greater than the AUL of the second superabsorbent particles, and wherein the first superabsorbent particles have an AUL of greater than 15 g/g, according to the test method herein; wherein the the first superabsorbent particles are comprised at a level of less than 80%wt, and the second superabsorbent particles (SAP2) are comprised at a level of at least 20%wt, by total weight of superabsorbent particles.

2. An absorbent core (1) according to Claim 1 wherein the central layer (4) comprises a void volume that is greater than the void volume of the fluid-permeable top layer, preferably wherein the top layer has an average pore size and/or void volume that are smaller than the average diameter of the superabsorbent particles.

3. An absorbent core (1) according to any of the preceding claims wherein the superabsorbent particles (9) are mechanically restrained and/or immobilized by a fiber network (10) of the high loft fibrous nonwoven layer.

4. An absorbent core (1) according to claim 3 wherein the high loft fibrous nonwoven layer has been subjected to a void volume decrease from a second void volume, generally corresponding with a superabsorbent particles deposition thereon, to a third void volume, generally corresponding to a state in which the superabsorbent particles have been deposited and impregnated thereon and/or therein, and wherein the third void volume is less, preferably at least 1.2 times less, even more preferably at least 1.5 times less, even more preferably at least 2 times less, than the second void volume.

5. An absorbent core (1) according to any of the preceding claims wherein the superabsorbent particles (9) are impregnated in a pattern comprising one or more areas substantially free of said superabsorbent particles arranged such to form one, two, or more longitudinally extending channels (11) substantially free of superabsorbent particles (9) wherein said channels extend substantially parallel to the longitudinal direction (y).

6. An absorbent core (1) according to claim 5 wherein the channels (11) are flanked and/or enclosed by superabsorbent particles (9) when viewed in a planar direction wherein the respective plane is formed by the transversal direction (x) and a longitudinal direction (y).

7. An absorbent core (1) according to any of the preceding claims wherein the fibers (10) of the high loft fibrous nonwoven layer comprise or consist of a material selected from the group consisting of: polylactic acid or derivatives thereof, polylactic-co-glycolic acid or derivatives thereof, polyhydroxyalkanoates or derivatives thereof, bio-polyethylene, bio-polypropylene, and mixtures thereof, preferably polylactic acid or derivatives thereof.

8. An absorbent core (1) according to any of the preceding claims wherein the central layer (4) is different from the top and bottom layers (2, 3), preferably wherein the top and bottom layers (2, 3) comprise, preferably consist of, a multilayer nonwoven comprising spunbond and/or meltblown fibers.

9. An absorbent core (1) according to claim 8 wherein the multilayer nonwoven is selected from the group consisting of: SS, SSS, SM, SMS, and SMMS.

10. An absorbent core (1) according to any of the preceding claims wherein the first superabsorbent particles (SAP1) have a particle size distribution that is greater than that of the second superabsorbent particles (SAP2).

11. An absorbent core (1) according to Claim 10 wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

12. An absorbent core (1) according to Claims 10 to 11 wherein first superabsorbent particles (SAP1) are comprised at a level of from 10%wt to 32%wt by total weight of superabsorbent particles; and the second superabsorbent particles (SAP2) are comprised at a level of from 25%wt to 100%wt, preferably from 30% to 90%, more preferably from 35% to 80%, even more preferably from 40% to 70%, even more preferably from 45% to 68%, by total weight of superabsorbent particles.

13. An absorbent core (1) according to Claims 10 to 12 wherein the AUL ratio AUL_{SAP1}/AUL_{SAP2}) of the first superabsorbent particles (SAP1) and second superabsorbent particles (SAP2) is greater than 1.4, preferably greater than 1.5, more preferably from 1.6 to 5, even more preferably from 1.7 to 3.

14. An absorbent core (1) according to any of the preceding Claims wherein the high loft fibrous nonwoven layer is free of hotmelt adhesive and preferably free of channels being substantially free of absorbent material within the deposition area (A_{D}), and preferably is substantially free of adhesive.

15. An absorbent core (1) according to any of the preceding Claims wherein the central layer(s) comprises from 20%wt to 60%wt of stiffening fibers, preferably having a linear density of about 1 dtex to about 6 dtex; and/or wherein the central layer(s) comprises stiffening fibers selected from bi-component fibers comprising polyethylene and polyethylene terephthalate components or polyethylene terephthalate and co-polyethylene terephthalate components.

16. An absorbent article (12) comprising a liquid permeable topsheet (13), a liquid impermeable backsheet (14) and an absorbent core (1) sandwiched therebetween, wherein the absorbent core (1) is according to any of the preceding Claims, and preferably further comprising an acquisition distribution layer (15) positioned between the topsheet (13) and the absorbent core (1), wherein said acquisition distribution layer (15) is a carded, spunbond and/or spunlaced nonwoven, preferably a carded air-through bonded nonwoven or spunlaced nonwoven; and/or wherein the acquisition distribution layer (15) has a basis weight of from 15 to 55 g/m2 and comprises fibres having an average diameter of from 10 to 35 microns, preferably from 15 to 30 microns, more preferably from 17 to 27 microns.

## Patentansprüche

1. Saugfähiger Kern (1) zur Verwendung in einem saugfähigen Artikel (12), wobei sich der saugfähige Kern (1) in einer Querrichtung (x) und einer Längsrichtung (y) erstreckt, wobei der saugfähige Kern (1) umfasst: eine flüssigkeitsdurchlässige Oberschicht (2); eine Unterschicht (3); eine oder mehrere Mittelschichten (4), die zwischen der Oberschicht (2) und der Unterschicht (3) sandwichartig angeordnet sind, wobei jede Mittelschicht eine Vorderkante (5), eine Hinterkante (6) und zwei sich in Längsrichtung erstreckende Seitenkanten (7, 8) aufweist, wobei die Mittelschicht (4) eine hochbauschige faserige Vliesschicht ist, die frei von Zellulosefasern ist; **dadurch gekennzeichnet, dass** die hochbauschige faserige Vliesschicht aus einem kardierten, luftdurchströmten Vliesstoff oder einem trockengelegten, thermisch gebundenen Vliesstoff besteht; und wobei die Mittelschicht (4) mit superabsorbierenden Partikeln (9) imprägniert ist, so dass die Partikel durch eine Dicke (z) der Mittelschicht hindurch enthalten sind, wobei sich die Dicke senkrecht sowohl zur Querrichtung (x) als auch zur Längsrichtung (y) erstreckt, und dass die hochbauschige faserige Vliesschicht im Wesentlichen frei von Schmelzklebstoff zur Immobilisierung der superabsorbierenden Partikel (9) ist, und wobei die superabsorbierenden Partikel eine Mischung aus superabsorbierenden Partikeln umfassen, die erste superabsorbierende Partikel und zweite superabsorbierende Partikel umfassen, wobei die ersten superabsorbierenden Partikel eine AUL aufweisen, die größer als die AUL der zweiten superabsorbierenden Partikel ist, und wobei die ersten superabsorbierenden Partikel eine AUL von mehr als 15 g/g gemäß dem hierin beschriebenen Testverfahren aufweisen; wobei die ersten superabsorbierenden Partikel in einem Anteil von weniger als 80 Gew.-% und die zweiten superabsorbierenden Partikel (SAP2) in einem Anteil von mindestens 20 Gew.-%, bezogen auf das Gesamtgewicht der superabsorbierenden Partikel, enthalten sind.

2. Saugfähiger Kern (1) nach Anspruch 1, wobei die Mittelschicht (4) ein Hohlraumvolumen aufweist, das größer ist als das Hohlraumvolumen der flüssigkeitsdurchlässigen Oberschicht, vorzugsweise wobei die Oberschicht eine durchschnittliche Porengröße und/oder ein Hohlraumvolumen aufweist, die kleiner sind als der durchschnittliche Durchmesser der superabsorbierenden Partikel.

3. Saugfähiger Kern (1) nach einem der vorhergehenden Ansprüche, wobei die superabsorbierenden Partikel (9) durch ein Fasernetzwerk (10) der hochbauschigen faserigen Vliesschicht mechanisch zurückgehalten und/oder immobilisiert sind.

4. Saugfähiger Kern (1) nach Anspruch 3, wobei die hochbauschige faserige Vliesschicht einer Hohlraumvolumenverringerung von einem zweiten Hohlraumvolumen, das im Allgemeinen einer Abscheidung von superabsorbierenden Partikeln darauf entspricht, zu einem dritten Hohlraumvolumen unterzogen wurde, das im Allgemeinen einem Zustand entspricht, in dem die superabsorbierenden Partikel darauf und/oder darin abgeschieden und imprägniert wurden, und wobei das dritte Hohlraumvolumen geringer ist, vorzugsweise mindestens 1,2-mal geringer, noch bevorzugter mindestens 1,5-mal geringer, noch bevorzugter mindestens 2-mal geringer, als das zweite Hohlraumvolumen.

5. Saugfähiger Kern (1) nach einem der vorhergehenden Ansprüche, wobei die superabsorbierenden Partikel (9) in einem Muster imprägniert sind, das einen oder mehrere Bereiche umfasst, die im Wesentlichen frei von den superabsorbierenden Partikeln sind, die so angeordnet sind, dass sie einen, zwei oder mehrere sich in Längsrichtung erstreckende Kanäle (11) bilden, die im Wesentlichen frei von superabsorbierenden Partikeln (9) sind, wobei sich die Kanäle im Wesentlichen parallel zur Längsrichtung (y) erstrecken.

6. Saugfähiger Kern (1) nach Anspruch 5, wobei die Kanäle (11) von superabsorbierenden Partikeln (9) flankiert und/oder umschlossen sind, wenn sie in einer ebenen Richtung betrachtet werden, wobei die jeweilige Ebene durch die Querrichtung (x) und die Längsrichtung (y) gebildet wird.

7. Saugfähiger Kern (1) nach einem der vorhergehenden Ansprüche, wobei die Fasern (10) der hochbauschigen faserigen Vliesschicht ein Material umfassen oder aus diesem bestehen, das ausgewählt ist aus der Gruppe bestehend aus: Polymilchsäure oder Derivaten davon, Polymilchsäure-co-Glykolsäure oder Derivaten davon, Polyhydroxyalkanoaten oder Derivaten davon, Bio-Polyethylen, Bio-Polypropylen und Mischungen davon, vorzugsweise Polymilchsäure oder Derivaten davon.

8. Saugfähiger Kern (1) nach einem der vorhergehenden Ansprüche, wobei sich die Mittelschicht (4) von der Oberschicht und der Unterschicht (2, 3) unterscheidet, vorzugsweise wobei die Oberschicht und die Unterschicht (2, 3) einen mehrschichtigen Vliesstoff umfassen, vorzugsweise aus diesem bestehen, der Spinnvlies- und/oder schmelzgeblasene Fasern umfasst.

9. Saugfähiger Kern (1) nach Anspruch 8, wobei der mehrschichtige Vliesstoff ausgewählt ist aus der Gruppe bestehend aus: SS, SSS, SM, SMS und SMMS.

10. Saugfähiger Kern (1) nach einem der vorhergehenden Ansprüche, wobei die ersten superabsorbierenden Partikel (SAP1) eine Partikelgrößenverteilung aufweisen, die größer ist als die der zweiten superabsorbierenden Partikel (SAP2).

11. Saugfähiger Kern (1) nach Anspruch 10, wobei zumindest die zweiten superabsorbierenden Partikel (SAP2) biobasierte superabsorbierende Kompositpolymerpartikel umfassen, vorzugsweise aus diesen bestehen, die ein synthetisches hydrophobes Polymer und ein natürliches Biopolymer umfassen, besonders bevorzugt aus einem Kompositpolymer zusammengesetzt sind, das Styrol-Maleinsäure-Copolymer und ein Biopolymer tierischen oder pflanzlichen Ursprungs umfasst; oder Nicht-Kompositpolymerpartikel, ausgewählt aus Polyacrylsäure, Natriumsalz, vernetzten, teilweise neutralisierten superabsorbierenden Partikeln.

12. Saugfähiger Kern (1) nach den Ansprüchen 10 bis 11, wobei die ersten superabsorbierenden Partikel (SAP1) in einem Anteil von 10 Gew.-% bis 32 Gew.-% bezogen auf das Gesamtgewicht der superabsorbierenden Partikel enthalten sind; und die zweiten superabsorbierenden Partikel (SAP2) in einem Anteil von 25 Gew.-% bis 100 Gew.-%, vorzugsweise von 30 % bis 90 %, besonders bevorzugt von 35 % bis 80 %, noch bevorzugter von 40 % bis 70 %, noch bevorzugter von 45 % bis 68 %, bezogen auf das Gesamtgewicht der superabsorbierenden Partikel, enthalten sind.

13. Saugfähiger Kern (1) nach den Ansprüchen 10 bis 12, wobei das AUL-Verhältnis (AULSAP1/AULSAP2) der ersten superabsorbierenden Partikel (SAP1) und der zweiten superabsorbierenden Partikel (SAP2) größer als 1,4 ist, vorzugsweise größer als 1,5, besonders bevorzugt von 1,6 bis 5, noch bevorzugter von 1,7 bis 3.

14. Saugfähiger Kern (1) nach einem der vorhergehenden Ansprüche, wobei die hochbauschige faserige Vliesschicht frei von Schmelzklebstoff und vorzugsweise frei von Kanälen ist, die im Wesentlichen frei von saugfähigem Material innerhalb des Abscheidungsbereichs (AD) sind, und vorzugsweise im Wesentlichen frei von Klebstoff ist.

15. Saugfähiger Kern (1) nach einem der vorhergehenden Ansprüche, wobei die Mittelschicht(en) von 20 Gew.-% bis 60 Gew.-% Versteifungsfasern umfassen, vorzugsweise mit einer linearen Dichte von etwa 1 dtex bis etwa 6 dtex; und/oder wobei die Mittelschicht(en) Versteifungsfasern umfassen, die ausgewählt sind aus Bikomponentenfasern, die Polyethylen- und Polyethylenterephthalat-Komponenten oder Polyethylenterephthalat- und Co-Polyethylenterephthalat-Komponenten umfassen.

16. Saugfähiger Artikel (12), umfassend eine flüssigkeitsdurchlässige Oberschicht (13), eine flüssigkeitsundurchlässige Unterschicht (14) und einen dazwischen angeordneten saugfähigen Kern (1), wobei der saugfähige Kern (1) einem der vorhergehenden Ansprüche entspricht, und vorzugsweise ferner eine Akquisitionsverteilungsschicht (15) umfasst, die zwischen der Oberschicht (13) und dem saugfähigen Kern (1) positioniert ist, wobei die Akquisitionsverteilungsschicht (15) ein kardiertes, spinngebundenes und/oder wasserstrahlverfestigtes Vlies ist, vorzugsweise ein kardiertes, luftdurchströmtes Vlies oder ein wasserstrahlverfestigtes Vlies; und/oder wobei die Akquisitionsverteilungsschicht (15) ein Flächengewicht von 15 bis 55 g/m2 aufweist und Fasern mit einem durchschnittlichen Durchmesser von 10 bis 35 Mikron, vorzugsweise von 15 bis 30 Mikron, besonders bevorzugt von 17 bis 27 Mikron, umfasst.

## Revendications

1. Âme absorbante (1) pour utilisation dans un article absorbant (12), l'âme absorbante (1) s'étendant dans une direction transversale (x) et une direction longitudinale (y), l'âme absorbante (1) comprenant : une couche supérieure perméable aux fluides (2) ; une couche inférieure (3) ; une ou plusieurs couches centrales (4) prises en sandwich entre la couche supérieure (2) et la couche inférieure (3), chaque couche centrale ayant un bord avant (5), un bord arrière (6) et deux bords latéraux s'étendant longitudinalement (7, 8), dans laquelle la couche centrale (4) est une couche non tissée fibreuse à grand volume qui est exempte de fibres de cellulose ; **caractérisée en ce que** ladite couche non tissée fibreuse à grand volume est constituée d'un non-tissé cardé lié par traversée d'air ou d'un non-tissé thermolié par voie sèche ; et dans laquelle ladite couche centrale (4) est imprégnée de particules superabsorbantes (9) de telle sorte que lesdites particules sont comprises à travers une épaisseur (z) de ladite couche centrale, ladite épaisseur s'étendant perpendiculairement à la fois à la direction transversale (x) et à la direction longitudinale (y), et **en ce que** ladite couche non tissée fibreuse à grand volume est sensiblement exempte d'adhésif thermofusible pour immobiliser lesdites particules superabsorbantes (9) et dans laquelle les particules superabsorbantes comprennent un mélange de particules superabsorbantes comprenant des premières particules superabsorbantes et des secondes particules superabsorbantes, dans laquelle les premières particules superabsorbantes ont une AUL qui est supérieure à l'AUL des secondes particules superabsorbantes, et dans laquelle les premières particules superabsorbantes ont une AUL supérieure à 15 g/g, selon la méthode d'essai décrite ici ; dans laquelle les premières particules superabsorbantes sont comprises à un niveau de moins de 80 % enpoids, et les secondes particules superabsorbantes (SAP2) sont comprises à un niveau d'au moins 20 % en poids, par rapport au poids total des particules superabsorbantes.

2. Âme absorbante (1) selon la revendication 1, dans laquelle la couche centrale (4) comprend un volume de vide qui est supérieur au volume de vide de la couche supérieure perméable aux fluides, de préférence dans laquelle la couche supérieure a une taille de pore moyenne et/ou un volume de vide qui sont inférieurs au diamètre moyen des particules superabsorbantes.

3. Âme absorbante (1) selon l'une quelconque des revendications précédentes, dans laquelle les particules superabsorbantes (9) sont retenues et/ou immobilisées mécaniquement par un réseau de fibres (10) de la couche non tissée fibreuse à grand volume.

4. Âme absorbante (1) selon la revendication 3, dans laquelle la couche non tissée fibreuse à grand volume a été soumise à une diminution de volume de vide d'un deuxième volume de vide, correspondant généralement à un dépôt de particules superabsorbantes sur celle-ci, à un troisième volume de vide, correspondant généralement à un état dans lequel les particules superabsorbantes ont été déposées et imprégnées sur et/ou dans celle-ci, et dans laquelle le troisième volume de vide est inférieur, de préférence au moins 1,2 fois inférieur, encore plus préférentiellement au moins 1,5 fois inférieur, encore plus préférentiellement au moins 2 fois inférieur, au deuxième volume de vide.

5. Âme absorbante (1) selon l'une quelconque des revendications précédentes, dans laquelle les particules superabsorbantes (9) sont imprégnées selon un motif comprenant une ou plusieurs zones sensiblement exemptes desdites particules superabsorbantes, agencées de manière à former un, deux, ou plusieurs canaux s'étendant longitudinalement (11) sensiblement exempts de particules superabsorbantes (9), dans laquelle lesdits canaux s'étendent sensiblement parallèlement à la direction longitudinale (y).

6. Âme absorbante (1) selon la revendication 5, dans laquelle les canaux (11) sont flanqués et/ou entourés par des particules superabsorbantes (9) lorsqu'on les regarde dans une direction planaire, dans laquelle le plan respectif est formé par la direction transversale (x) et la direction longitudinale (y).

7. Âme absorbante (1) selon l'une quelconque des revendications précédentes, dans laquelle les fibres (10) de la couche non tissée fibreuse à grand volume comprennent ou sont constituées d'un matériau choisi dans le groupe constitué par : l'acide polylactique ou ses dérivés, l'acide polylactique-co-glycolique ou ses dérivés, les polyhydroxyalcanoates ou leurs dérivés, le bio-polyéthylène, le bio-polypropylène, et leurs mélanges, de préférence l'acide polylactique ou ses dérivés.

8. Âme absorbante (1) selon l'une quelconque des revendications précédentes, dans laquelle la couche centrale (4) est différente des couches supérieure et inférieure (2, 3), de préférence dans laquelle les couches supérieure et inférieure (2, 3) comprennent, de préférence sont constituées de, un non-tissé multicouche comprenant des fibres filées-liées (spunbond) et/ou soufflées à l'état fondu (meltblown).

9. Âme absorbante (1) selon la revendication 8, dans laquelle le non-tissé multicouche est choisi dans le groupe constitué par : SS, SSS, SM, SMS, et SMMS.

10. Âme absorbante (1) selon l'une quelconque des revendications précédentes, dans laquelle les premières particules superabsorbantes (SAP1) ont une distribution de taille de particule qui est supérieure à celle des secondes particules superabsorbantes (SAP2).

11. Âme absorbante (1) selon la revendication 10, dans laquelle au moins les secondes particules superabsorbantes (SAP2) comprennent, de préférence sont constituées de, particules de polymère composite superabsorbant biosourcé comprenant un polymère hydrophobe synthétique et un biopolymère naturel, plus préférentiellement composées d'un polymère composite comprenant un copolymère d'acide styrène maléique et un biopolymère d'origine animale ou végétale ; ou des particules de polymère non composite choisies parmi l'acide polyacrylique, le sel de sodium, réticulées, des particules superabsorbantes partiellement neutralisées.

12. Âme absorbante (1) selon les revendications 10 à 11, dans laquelle les premières particules superabsorbantes (SAP1) sont comprises à un niveau de 10 % en poids à 32 % en poids par rapport au poids total des particules superabsorbantes ; et les secondes particules superabsorbantes (SAP2) sont comprises à un niveau de 25 % en poids à 100 % en poids, de préférence de 30 % à 90 %, plus préférentiellement de 35 % à 80 %, encore plus préférentiellement de 40 % à 70 %, encore plus préférentiellement de 45 % à 68 %, par rapport au poids total des particules superabsorbantes.

13. Âme absorbante (1) selon les revendications 10 à 12, dans laquelle le rapport d'AUL (AULSAP1/AULSAP2) des premières particules superabsorbantes (SAP1) et des secondes particules superabsorbantes (SAP2) est supérieur à 1,4, de préférence supérieur à 1,5, plus préférentiellement de 1,6 à 5, encore plus préférentiellement de 1,7 à 3.

14. Âme absorbante (1) selon l'une quelconque des revendications précédentes, dans laquelle la couche non tissée fibreuse à grand volume est exempte d'adhésif thermofusible et de préférence exempte de canaux étant sensiblement exempts de matériau absorbant à l'intérieur de la zone de dépôt (AD), et est de préférence sensiblement exempte d'adhésif.

15. Âme absorbante (1) selon l'une quelconque des revendications précédentes, dans laquelle la ou les couches centrales comprennent de 20 % en poids à 60 % en poids de fibres de renforcement, ayant de préférence une densité linéaire d'environ 1 dtex à environ 6 dtex ; et/ou dans laquelle la ou les couches centrales comprennent des fibres de renforcement choisies parmi des fibres bicomposant comprenant des composants de polyéthylène et de polyéthylène téréphtalate ou des composants de polyéthylène téréphtalate et de co-polyéthylène téréphtalate.

16. Article absorbant (12) comprenant une feuille de dessus perméable aux liquides (13), une feuille de fond imperméable aux liquides (14) et une âme absorbante (1) prise en sandwich entre elles, dans lequel l'âme absorbante (1) est conforme à l'une quelconque des revendications précédentes, et comprenant de préférence en outre une couche d'acquisition-distribution (15) positionnée entre la feuille de dessus (13) et l'âme absorbante (1), dans lequel ladite couche d'acquisition-distribution (15) est un non-tissé cardé, filé-lié (spunbond) et/ou hydrolié (spunlaced), de préférence un non-tissé cardé lié par traversée d'air ou un non-tissé hydrolié ; et/ou dans lequel la couche d'acquisition-distribution (15) a un grammage de 15 à 55 g/m2 et comprend des fibres ayant un diamètre moyen de 10 à 35 microns, de préférence de 15 à 30 microns, plus préférentiellement de 17 à 27 microns.
